# EUROPEAN PATENT APPLICATION

(11) **EP 3 574 826 A1**
(43) Date of publication of application: **04.12.2019**
(21) Application number: 19177055.1
(22) Date of filing: 28.05.2019
(51) Int. Cl.: A61B 5/053, A61B 5/00

(54) **TOUCH DETECTION BASED ON FREQUENCY RESPONSE OF TISSUE**

(30) Priority: 29.05.2018 US 201815991291
(71) Applicant: Biosense Webster (Israel) Ltd., Yokneam, 2066717 (IL)
(72) Inventor: GOVARI, Assaf, 2066717 Yokneam (IL)
(74) Representative: Small, Gary James

(57) **Abstract**

A method includes measuring a first impedance-magnitude between a distal-electrode fitted at a distal end of a medical instrument in an organ of a patient and a surface-electrode attached externally to the patient, at a first electrical frequency. A second impedance-magnitude between the distal-electrode and the surface-electrode, is measured at a second electrical frequency. A difference between the first and second measured impedance-magnitudes is calculated. Based on the calculated difference, an indication of, whether the distal-electrode is in physical contact with tissue or immersed in blood is decided, and outputted.

## Description

### FIELD OF THE INVENTION

The present invention relates generally to intrabody medical procedures and instruments, and particularly to cardiac electro-anatomical sensing and ablation.

### BACKGROUND OF THE INVENTION

Various techniques have been suggested for verifying catheter contact with cardiac tissue. For example, U.S. Patent Application Publication 2016/0287137 describes a method and system for assessing electrode-tissue contact before the delivery of ablation energy. The method may generally include determining a difference between a maximum impedance magnitude at a low frequency for a given electrode and an absolute minimum impedance magnitude at the low frequency across all electrodes, determining a difference between a maximum impedance magnitude at a high frequency for a given electrode and an absolute minimum impedance magnitude at the high frequency across all electrodes, and determining a difference between a maximum impedance phase at the high frequency for a given electrode and an absolute minimum impedance phase at the high frequency across all electrodes. These differences may be correlated to one another using a linear model, the results of which determining whether the given electrode is in contact or not in contact with tissue.

U.S. Patent Application Publication 2016/0278841 describes a medical instrument that comprises an elongate body having a proximal end and a distal end and a pair of electrodes or electrode portions, for example a split-tip electrode assembly. Systems and methods are described that perform contact sensing and/or ablation confirmation based on electrical measurements obtained while energy of different frequencies is applied to the pair of electrodes or electrode portions. The contact sensing systems and methods may calibrate network parameter measurements to compensate for a hardware unit in a network parameter measurement circuit or to account for differences in cables, instrumentation or hardware used.

### SUMMARY OF THE INVENTION

An embodiment of the present invention provides a method including measuring a first impedance-magnitude between a distal-electrode fitted at a distal end of a medical instrument in an organ of a patient and a surface-electrode attached externally to the patient, at a first electrical frequency. A second impedance-magnitude between the distal-electrode and the surface-electrode, is measured at a second electrical frequency. A difference between the first and second measured impedance-magnitudes is calculated. Based on the calculated difference, an indication of, whether the distal-electrode is in physical contact with tissue or immersed in blood is decided, and outputted.

In some embodiments, the method includes comparing the calculated difference to a given threshold, and deciding that the distal-electrode is in touch with tissue if the difference is larger than the given threshold.

In some embodiments, the method includes measuring at the first electrical frequency a first set of impedance-magnitudes and measuring at the second electrical frequency a second set of impedance-magnitudes, wherein calculating the difference comprises calculating multiple differences between the respective first and second impedance-magnitudes, and wherein deciding whether the distal-electrode is in physical contact with tissue comprises making a decision based on the multiple calculated differences.

In an embodiment, the method further includes measuring the first and second sets of impedance-magnitudes by a plurality of distal-electrodes.

In an embodiment, the method further includes fitting one or more statistical distributions to the multiple calculated differences. One or more statistical tests are applied to the distributions. Based on results of the one or more statistical tests, whether a calculated difference among the respective set of differences is statistically significant is deduced. Based on whether the calculated difference is statistically significant, whether the distal-electrode is in physical contact with tissue or immersed in blood is decided.

In some embodiments, the first electrical frequency equals 1.5KHz or lower, and wherein the second electrical frequency equals 20KHz or higher.

In some embodiments, the method includes denoting the indication on an electro-anatomical map.

There is additionally provided, in accordance with an embodiment of the present invention, a system, including an electrical interface and a processor. The electrical interface is configured for communicating with a distal-electrode fitted at a distal end of a medical instrument in an organ of a patient. The processor is configured to receive, via the electrical interface, (i) a first impedance-magnitude measured between the distal-electrode and a surface-electrode at a first electrical frequency, and (ii) a second impedance-magnitude measured between the distal-electrode and the surface-electrode at a second electrical frequency. The processor is further configured to calculate a difference between the first and second measured impedance-magnitudes, and based on the calculated difference, decide, and output an indication of, whether the distal-electrode is in physical contact with tissue or immersed in blood.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a system for electro-anatomical mapping, in accordance with an embodiment of the present invention;
Fig. 2 is a schematic graph illustrating the frequency dependence of the impedance-magnitude of cardiac tissue vs. blood, in accordance with an embodiment of the present invention; and
Fig. 3 is a flow chart that schematically illustrates a method for sensing touch with cardiac tissue, in accordance with an embodiment of the present invention.

### DETAILED DESCRIPTION OF EMBODIMENTS

### OVERVIEW

During a catheterization procedure of an organ of the body, such as cardiac electro-anatomical mapping and/or ablation, there might be a need to verify that an electrode fitted at a distal end of a medical instrument, such as a catheter, is in physical contact with tissue. In order to verify such physical contact, a contact force sensor at the distal end of the catheter may be used. The need to use a dedicated sensor, however, might cause the catheterization procedure to be cumbersome and time-consuming, and also increase the size and cost of the catheter.

Embodiments of the present invention that are described herein provide methods and systems for determining whether a distal-electrode of a catheter is in physical contact with tissue of an organ, such as cardiac tissue, by using the distal-electrode itself without additional hardware. In some cases, physical contact can be even verified using the same electrical signals that the electrode applies and/or senses. Moreover, in some cases the physical contact can be determined while the electrode is used for performing other tasks, such as impedance-based position measurement, electro-anatomical sensing of cardiac tissue and/or ablation.

For clarity and simplicity, the description that follows refers to a single distal-electrode. Alternatively, any suitable number of distal-electrodes may be fitted to a catheter, and there can be more than one catheter in the heart in parallel that employ the disclosed method.

To verify physical contact, embodiments of the present invention utilize measurements of the magnitude of the electrical bio-impedance between the distal-electrode and one or more body-surface electrodes. This magnitude is referred to hereinafter as 'impedance-magnitude.' The dependence of the impedance-magnitude on electrical frequency provides an indication of whether or not the distal-electrode is in direct physical contact (i.e., touches) the cardiac tissue.

Specifically, over a specified range of frequencies, e.g., between approximately 1kHz and 30kHz, the impedance-magnitude of cardiac tissue decreases sharply with frequency. The impedance-magnitude of blood, on the other hand, is largely independent of frequency. In some embodiments, a processor analyses the frequency-dependent difference in impedance-magnitude to determine whether the distal-electrode is in touch with tissue or is in blood. For determining contact, the processor may apply any suitable criterion, such as check whether the difference in impedance-magnitude exceeds a given threshold.

In some embodiments, the distal-electrode is used for injecting and/or measuring frequency dependent electrical signals (e.g., voltages and/or currents and/or impedances). When the electrode injects the signals, one or more surface-electrodes are used for measuring the frequency dependent signals. In alternative embodiments, voltages modulated at different frequencies are applied between the surface-electrodes, and the distal-electrode is used for measuring resulting signals. Either way, the processor analyzes the measured frequency-dependent signals, and determines whether the distal-electrode is in physical contact with cardiac tissue or immersed in blood.

In some embodiments, the processor performs statistical analysis of multiple measured frequency-dependent impedance-magnitudes, for providing robust indication of physical contact of the distal-electrode with tissue. The processor may apply any suitable statistical test (e.g., *t*-test), and provide the test-result. If a given indication per a given distal-electrode passes the statistical test as a statistically significant one, then the processor compares the indication to a criterion, such as a preset threshold of change in impedance-magnitude between given low and high frequencies, so as to determine whether the specific distal-electrode is in touch with tissue or immersed in blood.

In some embodiments, the disclosed technique is used in a catheter based electro-anatomical mapping and ablation system, which uses the distal-electrode for determining locations where arrhythmia may originate from or propagate through, for diagnostic purposes and/or for enabling a physician to decide whether to ablate selected locations on an inner surface of the heart.

The disclosed technique eliminates the need for dedicated sensors and other hardware for detecting physical contact of an electrode with tissue. Avoiding additional system resources and eliminating the need to package additional sensors into the limited space of a catheter distal end may simplify catheters and/or catheter-based systems. As a result, cardiac diagnostic and therapeutic systems can be simplified. The disclosed techniques may also simplify and further improve the clinical outcome of diagnostic and therapeutic procedures.

### SYSTEM DESCRIPTION

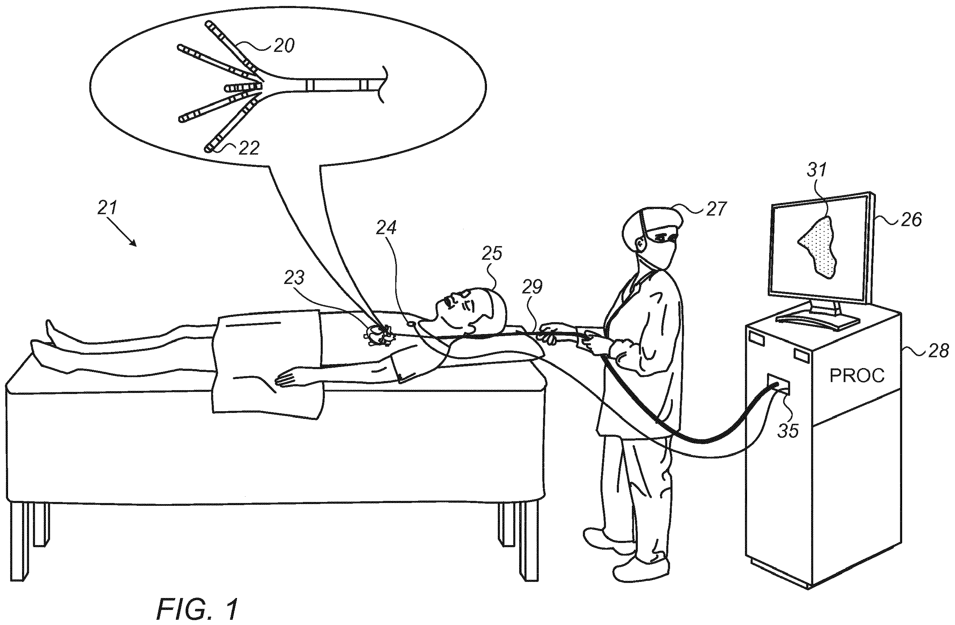

Fig. 1 is a schematic, pictorial illustration of an electro-anatomical mapping system, in accordance with an embodiment of the present invention. Fig. 1 depicts a physician 27 using an electro-anatomical catheter 29 to perform an electro-anatomical mapping of a heart 23 of a patient 25. Catheter 29 comprises, at its distal end, one or more arms 20, which may be mechanically flexible, to each of which are coupled one or more distal-electrodes 22. During the mapping procedure, electrodes 22 acquire and/or inject signals from and/or to the tissue of heart 23. A processor 28 receives these signals via an electrical interface 35, and uses information contained in these signals to construct an electro-anatomical map 31. During and/or following the procedure, processor 28 may display electro-anatomical map 31 on a display 26.

During the procedure, a tracking system is used to track the respective locations of distal-electrodes 22, such that each of the signals may be associated with the location at which the signal was acquired. For example, the Active Current Location (ACL) system, which is described in US Patent 8,456,182, whose disclosure is incorporated herein by reference, may be used. In the ACL system, a processor estimates the respective locations of the electrodes based on impedances measured between each of distal-electrodes 22 and a plurality of surface-electrodes 24 that are coupled to the skin of patient 25.

For example, three surface-electrodes 24 may be coupled to the patient's chest, and additional three surface-electrodes 24 may be coupled to the patient's back (For ease of illustration, only one surface-electrode is shown in Fig. 1). While electrodes 22 are inside heart 23 of the patient, electric currents are passed between electrodes 22 and surface-electrodes 24. Based on the ratios between the resulting current amplitudes measured at surface-electrodes 24 (or between the impedances implied by these amplitudes), and given the known positions of electrodes 24 on the patient's body, processor 28 calculates an estimated location of each of electrodes 22 within the patient's heart. The processor may thus associate any given impedance signal received from electrodes 22 with the location at which the signal was acquired.

In an embodiment, processor 28 is further configured to estimate and verify the quality of physical contact between each of electrodes 22 and an inner surface of cardiac chamber during measurement. The indication is based on modeling the frequency response of the impedances sensed by each of electrodes 22, which is different for blood and for tissue, and thus may serve as indication of physical contact, as elaborated below.

The example illustration shown in Fig. 1 is chosen purely for the sake of conceptual clarity. Impedance based measurements can also be done by applying voltage gradient using surface-electrodes 24 or other skin attached electrodes, use electrodes 22 for measuring voltages induced in the heart relative to a reference surface-electrode. One example of such a system is the Carto®4 technology produced by Biosense-Webster, Inc. (Irvine, California). Thus, embodiments of the present invention apply to any position sensing method in which electrodes apply and/or measure modulated electrical signals.

Other type of sensing and/or therapeutic catheters, such as the SmartTouch®, the Navistar® and the Lasso® Catheters (produced by Biosense-Webster, Inc.) may equivalently be employed. Contact sensors may be fitted at the distal end of electro-anatomical catheter 29. As noted above, other types of electrodes, such as those used for ablation, may be utilized in a similar way to electrodes 22 for acquiring the needed frequency dependent impedance data. Thus, an ablation electrode used for collecting frequency dependent impedance data is regarded for that matter in the description as a distal-electrode.

In general, processor 28 may be embodied as a single processor, or as a cooperatively networked or clustered set of processors. Processor 28 is typically a programmed digital computing device comprising a central processing unit (CPU), random access memory (RAM), non-volatile secondary storage, such as a hard drive or CD ROM drive, network interfaces, and/or peripheral devices. Program code, including software programs, and/or data are loaded into the RAM for execution and processing by the CPU and results are generated for display, output, transmittal, or storage, as is known in the art. The program code and/or data may be downloaded to the computer in electronic form, over a network, for example, or it may, alternatively or additionally, be provided and/or stored on non-transitory tangible media, such as magnetic, optical, or electronic memory. Such program code and/or data, when provided to the processor, produce a machine or special-purpose computer, configured to perform the tasks described herein.

### TOUCH DETECTION BY DIFFERENT FREQUENCY RESPONSE OF TISSUE

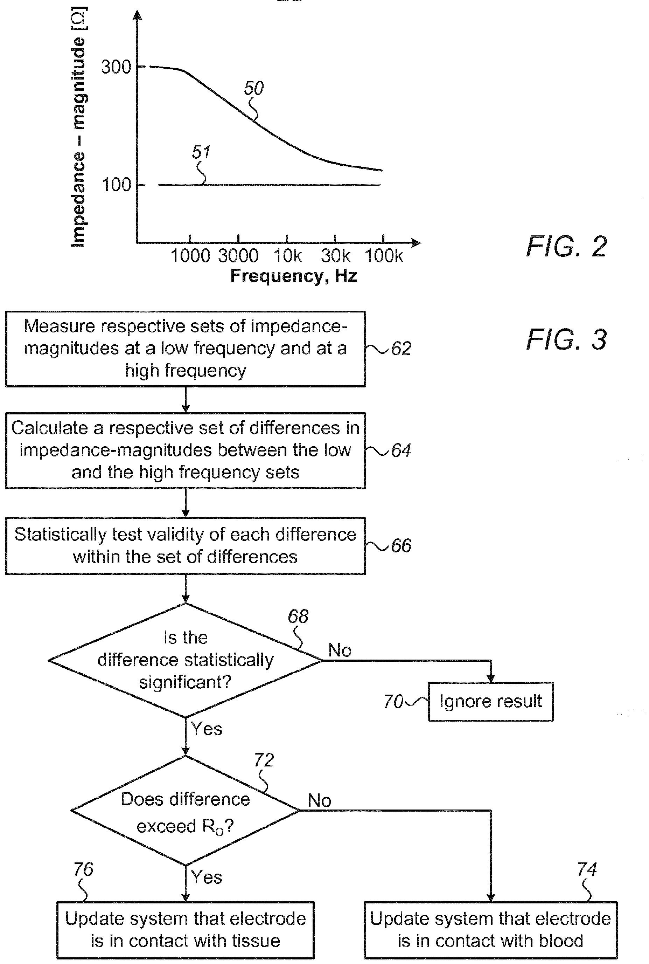

Fig. 2 is a schematic graph illustrating the frequency dependence of the impedance-magnitude of cardiac tissue vs. blood, in accordance with an embodiment of the present invention. The impedance-magnitude of cardiac tissue as a function of frequency is given by a curve 50. The impedance-magnitude of blood as a function of frequency is given by a curve 51.

The largely frequency independent blood impedance-magnitude depicted by curve 51 is about 100Ω. Cardiac tissue impedance-magnitude, as depicted by curve 50, is typically several times higher at the low end of frequencies (in some cases, approximately 300Ω), and becomes similar to blood at the higher end of frequencies. As seen, tissue impedance-magnitude is strongly frequency dependent approximately between 1kHz and 30kHz. Another example of a characterized frequency dependence of impedance-magnitude of blood vs. that of a body tissue is provided in "Medical Instrumentation: Application and Design," Webster (ed.) 3rd Ed., John Wiley & Sons, Inc., New-York, 1998.

Physiologically, impedance varies with frequency when the current flows through tissue because tissue cells permit passage of the high frequency (while absorbing some of its energy). In contrast, in blood plasma (fluid) conduction dominates, which is insensitive to the frequency. Consequently, if distal-electrode 22 is in good physical contact with cardiac tissue (as opposed to being immersed in blood), the resulting difference in impedance-magnitudes measured using that electrode (as a function of frequency) should be noticeable. Thus, in the case of using an ACL system, a change of frequency of an injected modulated current to tissue, from a given low frequency to a given high frequency will be manifested in an impedance-magnitude difference measurement, while not manifested when modulated current is injected in blood.

In an embodiment, using a distal-electrode 22j (an index j is used in case several such electrodes exist), ACL system 20 measures a set of impedance-magnitudes |*Zⱼₖ*(*ωₗ*)| and |*Zⱼₖ*(*ωₕ*)|, between distal-electrode j and a surface electrode. The impedance values *Zⱼₖ*(*ωₗ*) and *Zⱼₖ*(*ωₕ*) are complex values having both magnitudes and phases. The impedance-magnitudes |*Zⱼₖ*(*ωₗ*)| and |*Zⱼₖ*(*ωₕ*)| disregard the phase information.

The frequency *ωₗ* is a first, given low frequency, and frequency *ωₕ* is a second, given high frequency, of an injected modulated current. In an embodiment, the index *k* counts repeated measurements. In another embodiment, the index *k* belongs to one of surface-electrodes 24 (*k* = 1,2,...6).

As noted above, a difference in an impedance-magnitude, |Δ*Zⱼₖ*| = ∥*Zⱼₖ*(*ωₗ*)| - |*Zⱼₖ*(*ωₕ*)∥, may provide indication of the distal-electrode touching a tissue. Curves 50 and 51 of Fig. 2 suggest that when distal-electrode 22j is immersed in blood, then the corresponding differences in the impedance-magnitudes, |Δ*Zⱼₖ*(*B*)| = {∥*Zⱼₖ*(*ωₗ*)| - |*Zⱼₖ*(*ωₕ*)∥}*_{B},* will be generally smaller compared to those when distal-electrode 22j is in physical contact with tissue, |Δ*Zⱼₖ*(*T*)| = {∥*Zⱼₖ*(*ωₗ*)| - |*Zⱼₖ*(*ωₕ*)∥}*_{T}*.

In an embodiment, processor 28 compares the differences between impedance-magnitudes to a criterion, such as a given threshold having a value *R*₀, such that if a difference in impedance-magnitudes exceeds *R*₀ then the processor determines the distal-electrode is in contact with cardiac tissue.

In some cases, variations between differences |Δ*Zⱼₖ*(*B*)| and |Δ*Zⱼₖ*(*T*)| might be accounted for as elaborated above. However, in other cases, the different electrical path in the heart would account for only part of a total difference in impedances between distal-electrode 22j and a surface-electrode, for example, due to other variabilities in electrical paths in the body. Thus, differentiating |Δ*Zⱼₖ*(*T*)| from |Δ*Zⱼₖ*(*B*)|) in a meaningful way may not be a straightforward task.

In some embodiments, processor 28 performs statistical analysis of the numerous differences of impedance-magnitudes calculated from measurements, for providing robust indication of a physical contact of an electrode with tissue. For example, one or more statistical distributions (e.g., Normal distributions) may be fitted to the calculated impedance-magnitudes differences, such as {|Δ*Zⱼₖ*(*B*)| | *j* = 1,2, ... N; *k* = 1,2, ... 6} and {|Δ*Zⱼₖ*(*T*)||*j* = 1,2,*... N; k* = 1,2, ... 6}, for all *N* distal-electrodes 22 of the catheter and all six surface-electrodes.

The processor now applies a statistical test (e.g., t-test) to the distributions. In an embodiment, if a given difference |Δ*Zⱼₖ*| passes the statistical test as a statistically significant difference, then the processor compares the difference to a criterion, such as a threshold difference between impedance-magnitudes having a value *R*₀. If the calculated difference of impedance-magnitudes is below *R*₀, then the processor may update the system, for example by denoting in an electro-anatomical map, with the information that the specific distal-electrode is in touch with blood. If the calculated difference of impedance-magnitudes is above *R*₀, then the processor updates the system, with the information that the specific distal-electrode is in touch with tissue, for example by denoting that as a touched location on a map.

Using statistical approaches such as specified above may be specifically beneficial with multi-electrode catheters, a case in which additional statistical tools, such as correlations between impedance differences generated by a set of distal-electrodes, may be analyzed to provide indication of physical contact. Nevertheless, repeated measurement using a single distal electrode may as well increase the size of statistical ensembles in similar beneficial ways.

As Fig. 2 shows, the impedance-magnitude of tissue as a function of frequency varies mostly between about 1KHz and 30KHz. In an embodiment, the low frequency, *ωₗ*, equals 1.5KHz or less, while the high frequency, *ωₕ,* equals 20KHz or more. Selecting the low and high frequencies the way detailed above increases the robustness of impedance-magnitude measurements. Furthermore, robust measurements increase the validity of statistical tests to determine in a statistically significant manner if a calculated difference in impedance-magnitude is an indication of contact with tissue, an indication of contact with blood, or an insignificant result to be ignored. The numerical values of the high and low frequencies given above are chosen purely by way of example. In alternative embodiments, any other suitable frequencies values can be used.

The example shown in Fig. 2 is chosen purely for the sake of conceptual clarity. In another embodiment, for example with a Carto®4 based system, each distal-electrode, out of one or more distal-electrodes 22, senses modulated voltages that were applied between pairs of surface-electrodes 24, at a first frequency, *ωₗ*, and a second frequency, *ωₕ.* Therefore, the general touch detection principle described herein is applicable with electrodes either applying and/or sensing modulated electrical signals.

Fig. 3 is a flow chart that schematically illustrates a method for sensing physical touch between an electrode and cardiac tissue, in accordance with an embodiment of the present invention. Before the procedure may begin, physician 27 inserts electro-anatomical catheter 29 into the heart, deploys and engages tissue of heart 23 at a given location.

The process may then begin with processor 28 of system 20 measuring sets of impedance-magnitudes between distal-electrodes 22 and surface-electrodes 24 at low and high frequencies, at a measurement step 62. In one embodiment, as explained above with respect to Fig. 2, the low frequency is chosen below 1.5KHz, and the high frequency is chosen above 20KHz. Alternatively, any other suitable low and high frequencies can be used.

At a calculation step 64, processor 28 calculates, for each distal-electrode 22, an associated set of differences in impedance-magnitudes between impedance-magnitudes measured at low and high frequencies. Next, at a statistical testing step 66, processor 28 fits to all impedance-magnitude differences one or more statistical distributions and applies one or more statistical tests, to determine whether impedance-magnitude differences (associated each with an individual distal-electrode 22) are statistically significant.

If, at a checking step 68, the calculated difference is statistically insignificant, processor 28 drops the result (i.e., ignores it), at a dropping step 70. If the calculated difference is statistically significant, then processor 28 applies a suitable criterion, such as comparing the difference to a threshold having a value *R*₀, at a comparing step 72. If the calculated difference of impedance-magnitudes is below *R*₀, then processor 28 updates the system, at a blood decision step 74, with the information that the specific distal-electrode 22 is immersed in blood and not in touch with the cardiac tissue. If the calculated difference of impedance-magnitudes is above *R*₀, then processor 28 updates the system, at a tissue decision step 76, with the information that the specific distal-electrode 22 is in touch with cardiac tissue.

The procedure may be repeated several times for the same location, or move to another location on the inner surface of heart 23 by moving the catheter. The method may then return to step 62.

The example flow chart shown in Fig. 3 is chosen purely for the sake of conceptual clarity. In alternative embodiments, for example, various additional methods and/or sensors may be applied to assess physical contact with tissue. In alternative embodiments, statistical test step 66 may be omitted, for example if measurement results have a sufficiently large signal to noise ratio. The process then goes directly from step 64 to step 72.

Although the embodiments described herein mainly address pulmonary vein isolation, the methods and systems described herein can also be used in other cardiac applications.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A method, comprising:
measuring a first impedance-magnitude between a distal-electrode fitted at a distal end of a medical instrument in an organ of a patient and a surface-electrode attached externally to the patient, at a first electrical frequency;
measuring a second impedance-magnitude between the distal-electrode and the surface-electrode, at a second electrical frequency;
calculating a difference between the first and second measured impedance-magnitudes; and
based on the calculated difference, deciding, and outputting an indication of, whether the distal-electrode is in physical contact with tissue or immersed in blood.

2. The method according to claim 1, wherein deciding whether the distal-electrode is in physical contact with tissue comprises comparing the calculated difference to a given threshold, and deciding that the distal-electrode is in touch with tissue if the difference is larger than the given threshold.

3. The method according to claim 1, wherein measuring the first and second impedance-magnitudes comprises measuring at the first electrical frequency a first set of impedance-magnitudes and measuring at the second electrical frequency a second set of impedance-magnitudes, wherein calculating the difference comprises calculating multiple differences between the respective first and second impedance-magnitudes, and wherein deciding whether the distal-electrode is in physical contact with tissue comprises making a decision based on the multiple calculated differences.

4. The method according to claim 3, and comprising measuring the first and second sets of impedance-magnitudes by a plurality of distal-electrodes.

5. The method according to claim 3, and comprising:
fitting one or more statistical distributions to the multiple calculated differences;
applying one or more statistical tests to the distributions;
based on results of the one or more statistical tests, deducing whether a calculated difference among the respective set of differences is statistically significant; and
based on whether the calculated difference is statistically significant, deciding whether the distal-electrode is in physical contact with tissue or immersed in blood.

6. The method according to claim 1, wherein the first electrical frequency equals 1.5KHz or lower, and wherein the second electrical frequency equals 20KHz or higher.

7. The method according to claim 1, wherein outputting the indication comprises denoting the indication on an electro-anatomical map.

8. A system, comprising:
an electrical interface, for communicating with a distal-electrode fitted at a distal end of a medical instrument in an organ of a patient; and
a processor configured to:
receive, via the electrical interface, (i) a first impedance-magnitude measured between the distal-electrode and a surface-electrode at a first electrical frequency, and (ii) a second impedance-magnitude measured between the distal-electrode and the surface-electrode at a second electrical frequency;
calculate a difference between the first and second measured impedance-magnitudes; and
based on the calculated difference, decide, and output an indication of, whether the distal-electrode is in physical contact with tissue or immersed in blood.

9. The system according to claim 8, wherein the processor is configured to decide whether the distal-electrode is in physical contact with tissue by comparing the calculated difference to a given threshold, and deciding that the distal-electrode is in touch with tissue if the difference is larger than the given threshold.

10. The system according to claim 8, wherein the processor is configured to measure the first and second impedance-magnitudes by measuring at the first electrical frequency a first set of impedance-magnitudes and measuring at the second electrical frequency a second set of impedance-magnitudes, to calculate multiple differences between the respective first and second impedance-magnitudes, and to decide whether the distal-electrode is in physical contact with tissue based on the multiple calculated differences.

11. The system according to claim 10, wherein the processor is configured to measure the first and second sets of impedance-magnitudes by a plurality of distal-electrodes.

12. The system according to claim 10, wherein the processor is configured to:
fit one or more statistical distributions to the multiple calculated differences;
apply one or more statistical tests to the distributions;
based on results of the one or more statistical tests, deduce whether a calculated difference among the respective set of differences is statistically significant; and
based on whether the calculated difference is statistically significant, decide whether the distal-electrode is in physical contact with tissue or immersed in blood.

13. The system according to claim 8, wherein the first electrical frequency equals 1.5KHz or lower, and wherein the second electrical frequency equals 20KHz or higher.

14. The system according to claim 8, wherein the processor is configured to output the indication by denoting the indication on an electro-anatomical map.
